# FASCICULE DE BREVET EUROPEEN

(11) **EP 3 749 457 B1**
(45) Date de publication et mention de la délivrance du brevet: **07.06.2023**
(21) Numéro de dépôt: 19708587.1
(22) Date de dépôt: 05.02.2019
(51) Int. Cl.: B01L 9/00, B01L 3/00, A01N 1/02, A61B 10/00, G01N 1/00

(54) **DISPOSITIF DE STOCKAGE À TEMPÉRATURE AMBIANTE DE MATÉRIELS BIOLOGIQUES**
VORRICHTUNG ZUR LAGERUNG VON BIOLOGISCHEM MATERIAL BEI UMGEBUNGSTEMPERATUR
DEVICE FOR THE AMBIENT-TEMPERATURE STORAGE OF BIOLOGICAL MATERIAL

(30) Priorité: 05.02.2018 FR 1870121
(43) Date de publication de la demande: 16.12.2020
(73) Titulaire: L'etat Français Représenté Par Le Ministère De L'intérieur, 92130 Issy les Mulineaux (FR)
(72) Inventeur: HUBAC, Sylvain, 95000 Cergy (FR)
(74) Mandataire: Touroude, Magali Linda
(86) Numéro de dépôt international: PCT/FR2019/000016
(87) Numéro de publication internationale: WO 2019/150009

(56) Documents cités:
- EP-A1- 3 017 869
- WO-A1-2016/006363
- KR-B1- 100 996 967
- US-A1- 2004 265 187
- US-A1- 2007 116 613
- US-A1- 2010 248 213
- US-A1- 2015 266 019
- Thermofisher Scientific: "EasyStrip TM Plus, Ultra-Clear caps 0.2 mL (engineering diagram)", , 7 avril 2016 (2016-04-07), XP055505060, Extrait de l'Internet: URL:https://assets.thermofisher.com/TFS-As sets/LSG/manuals/AB2005_EngineeringDiagram .pdf [extrait le 2018-09-06]
- Imagene: "Imagene AutoStacker", , 21 septembre 2016 (2016-09-21), XP055505114, Parc Scientifique Unitec - 33600 PESSAC - FRANCE Extrait de l'Internet: URL:http://www.imagene.fr/fr/?s=autostacke r [extrait le 2018-09-06]

## Description

La présente invention concerne l'utilisation d'un dispositif de stockage à température ambiante de matériel biologique, comprenant au moins un récipient (1) constitué d'un fond (5) et d'une paroi latérale, ledit récipient (1) comprenant sur sa paroi latérale interne un moyen permettant de maintenir au-dessus et séparé du fond du récipient un support susceptible de contenir ledit matériel biologique à stocker. Le dispositif de stockage à température ambiante de matériel biologique peut également comprendre un assemblage (4) solidaire d'au moins 2, de préférence un multiple de 8, de manière plus préférée 96, 384, ou 1536 dispositifs selon l'invention, et un dispositif d'archivage de matériels biologiques, pouvant accueillir au moins 2 assemblages (4) selon l'invention. Enfin, la présente invention concerne une méthode de stockage de matériel biologique Le domaine de l'invention concerne en particulier le stockage et l'archivage de matériels biologiques.

À ce jour, les dispositifs permettant le stockage de quelques milliers à plusieurs millions d'échantillons de nature biologique sont de deux types. Ceux permettant leur stockage à des températures négatives et ceux permettant leur stockage à température ambiante.

Le stockage des échantillons à des températures négatives pouvant atteindre les conditions de congélation voir de cryogénisation nécessite des infrastructures complexes, coûteuses à contrôler et à maintenir en service et dont la mise en oeuvre nécessite l'emploi de personnels qualifiés notamment dans l'application de procédures strictes afin de prévenir tous risques d'incidents pouvant altérer l'intégrité des personnes et des échantillons. De plus, suivant le format du récipient dans lequel sont conditionnés de manière unitaire les échantillons (tubes 5-50 ml, microtubes 0,1-2 ml) l'espace nécessaire pour la conservation de plusieurs milliers à plusieurs millions d'échantillons est également un facteur pouvant devenir limitant dans le choix de ce mode de stockage. Afin de faciliter leur préparation et réduire la superficie des zones de stockage, les échantillons peuvent être conditionnés dans des microplaques de format compatible au format international SBS (Society of Biomolecular Screening) pouvant accueillir 96, 384 ou 1536 échantillons. Dans ce format, les échantillons d'une même microplaque, bien qu'individualisés dans un emplacement distinct, sont solidaires les uns des autres. En cas de nécessité de réutiliser un échantillon parmi les autres, tous les échantillons doivent ainsi subir l'étape de décongélation afin de permettre de récupérer l'échantillon d'intérêt puis de recongélation afin de poursuivre leur préservation. Ces étapes successives de congélation, décongélation sont de nature à dégrader les échantillons. Dans le but de préserver leur intégrité, le transport de ces échantillons doit également être réalisé dans les mêmes conditions que leur stockage.

Le stockage des échantillons à température ambiante permet d'éliminer les contraintes de dégradation des échantillons par les étapes successives de congélation/décongélation, ne nécessite pas d'infrastructures complexes à mettre en oeuvre et à entretenir. Selon ce mode de conservation, les frais de transport sont également diminués. On peut ainsi estimer que les frais de transport d'un même échantillon à une température négative sont environ 5 fois plus élevés qu'à température ambiante. En revanche, le processus technique de préparation des échantillons pour permettre leur stockage peut lui s'avérer assez complexe et peut nécessiter les services extérieurs de plateformes industrielles disposant du savoir-faire. C'est notamment le cas des services que peut proposer la société Imagene (France) dans son procédé de conservation de longue durée de molécules d'ADN (brevet EP 1 075 515 B1 (PCT/FR1998/000912 du 06/05/1998)).

Dans ce procédé industriel, l'ADN est déshydraté puis conservé dans une capsule métallique, appelée DNAshell^{®}, de 0,7 cm³, anhydre, anoxique et étanche. Il est possible de conserver ainsi plus de 60000 échantillons sur une superficie d'environ 1 m² et une hauteur d'environ 30 cm soit environ 400 000 échantillons pour 2 m³ (1 m² de surface au sol et 2 m de haut). D'autres solutions techniques beaucoup plus simples à mettre en oeuvre sont également présentes sur le marché. Il s'agit notamment des solutions DNAStable^{®} et DNAStable^{®} LD proposées par la société Biomatricia (USA) (demande de brevet US 20110081363 du 11/05/2010) ou de la solution GenTegra-DNA^{®} (WO2003040991 A2, US 7142987 B2, US 7584240 B2 GenVault Corporation) proposée par la société GenTegra qui permettent de conserver l'ADN sous forme déshydratée. Ces solutions peuvent ainsi être contenues dans des microtubes unitaires de 0,5 ou 1,7 ml dotés ou pouvant supporter un bouchon mais également dans des microtubes unitaires de 0,3 ou 0,75 ml pouvant être insérés dans une microplaque à 96 positions. Dans ce format de microplaque 96, les microtubes peuvent supporter un bouchon individuel mais plus généralement tous les échantillons sont rendus solidaires entre eux à la fin du processus de conservation par l'apposition d'un film afin d'assurer l'étanchéité des microtubes. Le procédé de conservation des échantillons sous forme déshydratée est composé de cinq étapes :
1. ajout d'un volume d'ADN sur la solution DNAStable^{®} ou GenTegra-DNA^{®} ;
2. homogénéisation de la solution à l'aide d'une micropipette ;
3. séchage pendant 4 à 72 heures à température ambiante ou 10 à 75 minutes à l'aide d'un concentrateur en fonction du volume d'ADN à déshydrater ;
4. fermeture du microtube ou scellage de la microplaque ;
5. conditionnement dans une cabine de séchage permettant de maintenir une hygrométrie < 40% ou conditionnement dans une pochette contenant des dessicants afin d'éviter la réhydratation de l'échantillon.

La solution DNAStable^{®} LD est conditionnée quant à elle sous forme liquide. Le procédé de conservation des échantillons d'ADN à l'aide de cette solution est composé également de cinq étapes :
1. ajout de 20µL de DNAStable^{®} LD à 1-100µl d'ADN ;
2. homogénéisation de la solution à l'aide d'une micropipette ;
3. séchage pendant 4 à 72 heures à température ambiante ou 10 à 75 minutes à l'aide d'un concentrateur en fonction du volume d'ADN à déshydrater ;
4. fermeture du microtube ou scellage de la microplaque ;
5. conditionnement dans une cabine de séchage permettant de maintenir une hygrométrie < 40% ou conditionnement dans une pochette contenant des dessicants afin d'éviter la réhydratation de l'échantillon.

À titre de comparaison avec 1a solution DNAshell^{®} de la société Imagene, la solution DNAStable^{®} au format microplaque 96 permettrait de stocker environ 57000 échantillons par m² sur une hauteur d'environ 30 cm soit environ 380 000 échantillons pour 2 m³ (1m² de surface au sol et 2 m de haut). L'échantillon d'ADN ainsi conservé par les dispositifs DNAshell^{®} (Imagene), DNAStable^{®}/DNAstable^{®} LD (Biomatricia) ou GenTegra-DNA^{®} (GenTegra) peut être réutilisé par simple réhydratation à l'eau.

Un autre procédé de conservation d'ADN à température ambiante encore plus simple à mettre en oeuvre est disponible sous la forme de cartes de prélèvements. Il s'agit par exemple des cartes FTA^{®} (demande de brevet n° WO 1996039813 du 07 juin 1996) ou FTA^{®} elute de la société General Electric (GE) (USA) et des cartes Nucleicard (demande de brevet n°WO 2015019205 du 07 août 2013) de la société COPAN (Italie). À titre d'exemple, le procédé de conservation des échantillons d'ADN à l'aide de la solution FTA^{®} Elute est composé de trois étapes :
1. dépôt de 25µL d'ADN sur la carte. Dans le cas de l'utilisation de cartes colorées, ce dépôt entraîne une décoloration de la carte permettant ainsi de favoriser la visualisation de la zone de dépôt ;
2. séchage à température ambiante pendant 3 heures ;
3. conditionnement de la carte dans une pochette contenant des dessicants afin d'éviter la réhydratation de l'échantillon.

Cependant, bien que certaines de ces cartes soient d'un format permettant leur manipulation de manière automatisée, leur taille importante de l'ordre de 45 cm² (9 cm x 5 cm) dans ce format nécessite un espace de stockage significatif. En effet, à l'issue de leur utilisation, ces cartes sont disposées dans des portoirs non hermétiques de dimensions incluant le couvercle de l'ordre de 12,5cm L x 9 cm 1 x 6 cm H permettant de loger jusqu'à 20 cartes. On peut ainsi estimer qu'il est possible de stocker environ 7200 échantillons par m² sur une hauteur d'environ 30 cm soit environ 48000 échantillons pour 2 m³ (1 m² de surface au sol et 2 m de haut). Bien que ces cartes présentes des propriétés de préservation de l'intégrité des biomolécules dans le temps, leur conservation à température ambiante et à pression atmosphérique est de nature à les exposer aux rayonnements UV ainsi qu'à l'oxygène de l'air qui constituent les principaux facteurs de dégradation biologique de l'ADN. Pour pallier à ces effets et préserver ainsi l'intégrité des prélèvements dans le temps, la société GE préconise de stocker ces cartes individuellement dans des enveloppes hermétiques avec la présence d'un dessicant afin de piéger l'humidité de l'air ambiant. Ces enveloppes sont de deux formats : 9,5 x 7,5 cm (71,25 cm²) (référence WB100036) ou 11 x 16,5 cm (181,5 cm²) (référence WB100036) suivant le format de la carte de prélèvement à stocker. Ce mode de conditionnement s'avère d'autant plus volumineux et contraignant. L'échantillon d'ADN ainsi conservé par le procédé FTA^{®} Elute peut être réutilisé selon un protocole en trois étapes :
1. découpe de la zone de dépôt l'aide d'un emporte-pièce dont la forme est généralement cylindrique. Cette opération peut être réalisée manuellement ou automatiquement à l'aide d'automates de poinçonnage. En revanche ce mode de prélèvement à l'aide d'un emporte-pièce ne permet pas de prélever l'intégralité de la superficie de la zone de décoloration dont la forme peut être variable. On peut ainsi estimer que seulement 40% de la zone de dépôt peut être prélevée ainsi. Ce procédé est également source de contaminations entre les échantillons par l'utilisation successive d'un même poinçon pour prélever plusieurs échantillons distincts. Pour prélever l'intégralité de la zone de dépôy, une solution, mais qui s'avérerait longue et fastidieuse, consisterait à découper manuellement, à l'aide d'un objet tranchant de type scalpel, l'intégralité de la zone de dépôt en s'adaptant à sa forme ;
2. remise en suspension de l'ADN dans une solution tampon ;
3. centrifugation pour séparer le support de l'ADN élué.

Dans le cas de liquides biologiques tels que le sang ou la salive directement déposés sur carte FTA^{®}, l'ADN présent est rendu directement disponible pour des applications de biologie moléculaire. L'échantillon d'ADN peut être ainsi réanalysé sans étape intermédiaire après la découpe d'un fragment de la zone décolorée de la carte.

Une solution alternative développée par la société GenTegra consiste à proposer une microplaque de 384 positions, appelée GenPlate^{®}, dont chacun des 384 puits contient un disque de papier FTA^{®} de 6 mm de diamètre. Ce procédé évite l'étape de poinçonnage de la carte FTA^{®} telle que décrit précédemment et supprime donc le risque de contamination des échantillons à cette étape. Le procédé de conservation est composé de trois étapes :
- dépôt de 10µl de liquide biologique (comme par exemple du sang) ou de 10µl d'une solution d'ADN sur le disque de papier FTA^{®} ;
- séchage pendant 12 à 16 heures à température ambiante ;
- apposition d'un film pour assurer l'étanchéité des échantillons.

L'échantillon d'ADN ainsi conservé par le procédé GenPlate^{®}, peut être réutilisé selon un protocole en trois étapes :
1. prélèvement du disque de papier FTA^{®} du puits de la microplaque GenPlate^{®} : 100% de la zone de dépôt est utilisée pour l'analyse ultérieure de l'échantillon contrairement aux 40% du protocole utilisant une carte FTA^{®} ;
2. remise en suspension de l'ADN dans une solution tampon (GenSolve DNA COMPLETE^{™} ;
3. centrifugation pour séparer le support de l'ADN élué.

De part la position des disques FTA^{®} en contact direct avec le fond du puits de la microplaque, une partie du liquide biologique déposé sur le disque traverse le papier et se retrouve transféré par contact au fond du puits. Cet inconvénient s'avère d'autant plus critique lorsque la quantité de matériel biologique déposée sur le disque est déjà limitante pour permettre une nouvelle analyse de l'échantillon.

En complément de la contrainte d'encombrement liée aux modes de stockage, se rajoute la contrainte de l'archivage qui doit permettre de pouvoir identifier le plus rapidement et le plus simplement possible l'emplacement d'un échantillon parmi des milliers voir plusieurs millions d'autres. A ce jour, le moyen utilisé pour identifier les cartes, les capsules DNAshell^{®} ou les microtubes /microplaques DNAStable^{®} ou GenTegra^{®} sont des identifiants « dits passifs » alphanumériques ou 2D. Est appelé ici identifiant passif un identifiant qui doit être lu de manière individuelle et en contact visuellement ou à l'aide d'un scanner contrairement à un identifiant actif de type puce RFID (Radio Frequency Identification) qui permet une localisation à distance d'un échantillon ou de plusieurs échantillons simultanément.

Concernant les échantillons de nature biologique et plus particulièrement les échantillons d'ADN prélevés dans le domaine de la criminalistique, les pratiques de prélèvement de ces échantillons, appelés traces biologiques, doivent permettre de conserver une partie de la trace de question pour des analyses ultérieures ou contradictoires (contre-analyse). En revanche, lorsque la quantité d'ADN présente sur la trace à prélever pourrait s'avérer insuffisante en vue d'obtenir un résultat exploitable, c'est-à-dire entre 50 et 100 picogrammes (pg) d'ADN, l'intégralité de la trace est alors prélevée afin de maximiser le succès de l'analyse. Dans ce cas, le seul moyen de procéder à une contre-analyse est d'analyser l'ADN résiduel qui se doit donc d'être conservé dans les meilleures conditions afin qu'il ne soit pas altéré. En raison du développement des biobanques, de la démocratisation des analyses ADN, du développement exponentiel des bases de données génétiques nationales à travers le monde et des différentes législations encadrant les modalités de conservation des traces biologiques en vue d'une nouvelle analyse, le besoin de conservation des échantillons biologiques à l'issue des analyses et plus particulièrement de l'ADN résiduel est estimé par an dans le monde à plusieurs centaines de milliers.

En outre, le document US2007/116613 présente un moyen de maintien du support constitué d'un épaulement qui ne permet pas à lui seul de maintenir le matériel biologique à stocker puisqu'il est systématiquement couplé à un système de serrage qui vient reposer sur l'épaulement permettant le maintien dudit matériel biologique dans le tube. Ce système encourage pour fixer le matériel biologique un accroissement de 1a surface de contact entre le dispositif et le matériel biologique présent sur le support non biologique, ce qui est préjudiciable en termes de contamination.

Le document KR 100 996 967 divulgue un récipient comprenant un anneau externe au récipient disposé sur des protubérances (E) pour être fixé à une hauteur donnée sur la paroi interne du récipient.

Le document WO 2016/006363 concerne de manière générale un analyseur automatique destiné à analyser automatiquement des composants comme le sang, comprenant un dispositif avec un épaulement permettant de supprimer le phénomène de capillarité se produisant dans l'espace entre la sonde de distribution et le récipient de réaction. Ainsi, il existe aujourd'hui un besoin pour des dispositifs permettant le stockage rapide de plusieurs milliers voir millions d'échantillons biologiques dans un minimum d'espace et avec un minimum de contraintes d'ordre logistique et technique pour permettre leur préservation dans le temps mais également leur réanalyse individuelle à moindre coût. Afin d'être pleinement efficace, ces dispositifs doivent également permettre d'assurer une traçabilité des échantillons pour être en mesure facilement et rapidement de localiser, transmettre et analyser à nouveau un échantillon d'intérêt sans nuire à la préservation des autres échantillons.

Aujourd'hui la Demanderesse innove dans le stockage et l'archivage de matériels biologiques, en proposant un nouveau dispositif permettant de répondre à ces besoins ; l'invention étant définie par les revendications jointes.

En effet, la Demanderesse a mis au point un dispositif de stockage de matériel biologique à température ambiante, comprenant un moyen permettant de maintenir le matériel biologique, pouvant être contenu dans des supports non biologiques, au-dessus du fond du dispositif de stockage, afin d'empêcher le transfert de matières biologiques présentes sur ces supports non biologiques, avec le fond du dispositif. Ledit dispositif est dans un mode de réalisation particulièrement préféré selon l'invention recouvert par un couvercle composé du même matériau permettant une étanchéité par simple pression sur le dispositif, et contient un identifiant unique passif de type Datamatrix 2D ou 3D et/ou un identifiant unique actif de type puce RFID. En outre, l'assemblage solidaire de 96, 384, ou 1536 dispositifs selon l'invention et leur archivage dans un dispositif décrit selon l'invention permet de réduire de façon conséquente les espaces de stockage nécessaires.

Ainsi, selon un premier objet, la présente invention concerne l'utilisation d'un dispositif pour le stockage à température ambiante de matériel biologique, comprenant au moins un récipient (1) constitué d'un fond (5) et d'une paroi latérale, ledit récipient (1) comprenant sur sa paroi latérale interne un moyen permettant de maintenir au-dessus et séparé du fond (5) du récipient (1) un support susceptible de contenir ledit matériel biologique à stocker.

Par « matériel biologique », on entend désigner selon la présente invention tout matériel issu d'un être vivant ou mort, pouvant se trouver sous forme de molécules, d'organites, de fluides, de fragments cellulaires ou de cellules, et présent sur un support biologique ou non biologique. De manière préférée selon l'invention, ledit matériel biologique à stocker contient des acides nucléiques, ADN et/ou ARN.

De manière préférée selon l'invention, ledit récipient (1) est de forme cylindrique.

De manière préférée selon l'invention, ledit récipient (1) a un volume compris entre environ 0,05 et environ 0,5 cm³ correspondant a minima à un récipient de forme cylindrique de 6,6 mm de diamètre sur 1,5 mm de hauteur.

De manière préférée selon l'invention, ledit support susceptible de contenir ledit matériel biologique à stocker est un support synthétique ou biologique.

Par « support biologique », on entend un échantillon biologique, pouvant être issu de la totalité ou partie d'un organisme vivant ou mort au moment de la collecte, par exemple issu d'un mammifère, de préférence d'origine humaine.

Par « support synthétique » ou non-biologique, on entend désigner selon la présente invention une surface non vivante telle que celle d'un objet ou d'une structure inerte. Il peut s'agir de tous types de matière comme des matières en fibres textiles, du coton, des coupes histologiques de paraffine, du nylon ou du papier à cigarettes. Il peut également s'agir de réactifs de type DNAStable^{®}, DNAStable^{®} LD, GenTegra-DNA^{®} ou tout autres types de réactifs présentant des propriétés physico-chimiques identiques ou équivalentes pour préserver l'ADN ou l'ARN dans le temps à température ambiante sous une forme déshydratée. En outre, il peut s'agir de fragments de cartes FTA^{®} ou tout autres types de support solide présentant des propriétés physico-chimiques identiques ou équivalents pour préserver l'ADN ou l'ARN dans le temps à température ambiante.

De manière préférée selon l'invention, ledit récipient (1) est en plastique ou en métal.

Par « plastique » est entendu un mélange contenant un polymère de base susceptible d'être moulé afin de conduire à un produit ou un objet. Cette dénomination contient trois familles de matières plastiques, les thermoplastiques, les thermodurcissables et les plastiques techniques. La famille des thermoplastiques comprend différents types de composés comme les Acrylonitrile butadiène styrène (Afcoryl, Bayblend, Cycolac, Isopak, Lastilac, Lustran, Novodur, Polyflam, Polylac, Polyman, Ronfalin, Terluran, Toyolac, Ugikral, Vestodur), l'acétate de cellulose (Cellidor A, Cellon, Lumarith, Rhodialite, Rhodoïd, Setilitte, Trialithe), les polystyrènes expansés (Afcolène, Depron, Hostapor, Polyfoam, Roofmate, Sagex, Styrocell, Styrodur, Styrofoam, Styropor, Vestypor), les polyamides (Akulon, Altech, Amilan, Bergamid, Capron, DuraForm, Durethan, Eratlon, Ertalon, Grilamid, Grilon, Igamid, Kevlar, Latamid, Lauramid, Maranyl, Minlon, Miramid, Nomex, Nylatron, nylon, Nypel, Orgamide, Perlon, Polyloy, Radiflam, Radilon, Renyl, Rilsan, Schulamid, Sniamid, Stanyl, Staramide, Starflam, Tactel, Technyl, Trogamid, Ultramid, Versamid, Vestamid, Vydyne, Zytel), les polybutykènes téréphtalates (Arnite, Celanex, Crastin, Deroton, Hostadur, Pocan, PTMT, Tenite, Ultradur, Vestodur), les polycarbonates (Apec, Axxis, Durolon, Gerpalon, Latilon, Lexan, Makrolon, Panlite, Plaslube, Polyman, Sunglass, Tuffak, Xantar), les polyéthylènes (Alkathène, Alketh, Dyneema, Eltex, Hostalen, Lacqtène, Lupolen, Manolène, Marlex, Moplen, Plastazote, Polythen, Sclair, Stamylan, Stamylex, Supralen, Surlyn, Tupperware, Tyvek, Vestolen A), les polyéthylènes terephtalates (Arnite, Baydur, Bidim, Dacron, Diolen, Ektar, Ertalyte, Hostadur K et A, Kodar, Mélinex, Mylar, Pocan, Raditer, Rhodester, Rynite, Tenite, Tergal, Terphane, Terylene, Trevira, Ultradur), les polyméthacrylates de méthyle (Acrigel, Altuglas, Altulite, Bonoplex, Corian, Deglan, Limacryl, Lucite, Metacrilat, Oroglas, Perspex, Plexiglas, Resalit, Vitroflex), les polyoxyméthylènes (Acetaver, Bergaform, Celcon, Delrin, Ertacetal, Hostaform, Kematal, Kepital, Kocetal, Ultraform), les polypropylènes (Amoco, Appryl, Carlona, Eltex, Hostalen PP, Luparen, Moplen, Novolen, Oléform, Polyflam, Profax, Propathene, Prylène, Stamylan P, Trovidur PP, Vestolen P), les polystyrènes (Carinex, Edistir, Empera, Gedex, Hostyrène, Lacqrène, Luran, Lustran, Lustrex, Noryl, Polyflam, Polystyrol, Riviera, Styranex, Styroflex, Styron, Trolitul, Ursaa, Vestyron), les polyacétates de vinyle (Elvacet, Hostaflex, Mowilith, Rhovyl, Vinnapas, Vinyon), les polychlorures de vinyle (Benvic, Breon, Corfam, Darvic, Dynel, Garbel, Gedevyl, Hostalit, Lacovyl, Lacqvil, Lucolène, Lucovyl, Lucalor, Lucoflex, Micronyl, Mipolam, Nakan, Saran, skaï, Solvic, Tefanyl, Trovidur, Ultryl, Vestolit, Vinidur, Vinnol, Vinnolit, Vinoflex, Vinylite), les styrènes-acrylonitriles (Cifra, Elvan, Kostil, Lacqsan, Luran, Lustran, Restil, Tyril, Vestoran). La famille des thermodurcissables comprend différents types de composés comme les polyépoxydes (Araldite, Devcon, DER, Doroxin, Epikote, Epon, Epotek, Epotuf, Epoxin, Eurepox, Lekutherm, Lopox, Rutapox), les mélamines (Arborite, Formica, Hostaset MF, Melochem, Melopas), les phénoplastes (Bakélite, Cascophen, toile bakélisée, papier bakélisé, bois bakélisé, Fluosite, Hostaset PF, Luphen, Micarta, Peracite, Trolitan, Tufnol), les polyuréthanes (Baydur, Bayflex, Baygal, Cyanapren, Daltoflex, Definal, Desmodur, Desmolin, Estolan, Lupranat, Lupranol, Luvipren, Moltopren, Napiol, Scurane, Urepan, Voranol, Vulkolian, Vulkollan), les aminoplastes (Aerodux, Beckamin, Cascamite, Hostaset UF, Pollopas, Prystal, Urochem) et les polyesters (Hostaset UP, Leguval, Palatal, Pregmat, Ukapon, Vestopol). Enfin la famille des plastiques techniques contient essentiellement des polytétrafluoroéthylènes (Algoflon, Ertaflon, Fluon, Gaflon, Halon, Hostaflon, Polyflon, Soreflon, Téflon, Voltalef).

Selon l'invention, ledit moyen permettant de maintenir le support susceptible de contenir ledit matériel biologique à stocker au-dessus et séparé du fond (5) du récipient (1) est un anneau (3) en métal ou en plastique.

Selon l'invention, ledit anneau (3) est moulé avec le récipient (1).

De manière préférée selon l'invention, ledit support susceptible de contenir ledit matériel biologique à stocker est choisi parmi les supports constitué de matière choisie parmi les fibres textiles, les plastiques, le papier, la paraffine utilisée pour des coupes histologiques dans le domaine de l'anatomo-pathologie, de préférence du papier traité chimiquement pour permettre la préservation dans le temps et à température ambiante de l'ADN extrait d'un échantillon biologique ou contenu dans un échantillon biologique.

Par « fibres textiles » est entendu autant les fibres textiles naturelles que chimiques, que ces dernières soient artificielles ou synthétiques. Ainsi, les fibres textiles naturelles végétales comprennent les fibres de chanvre, de Jute, de Kenaf, de lin, de lotus, d'orties, de ramie, de Trichostigma octandrum, d'abaca, d'alfa, d'ananas, d'asclepias, de bambou, de Bromelia karatas, de coton, de coco, de lin de Nouvelle-Zélande, de kapok, de papayer, de papyrus, de Sansevieria trifasciata, de sisal, et de yucca. Les fibres textiles naturelles animales comprennent les fibres d'alpaga, angora, de cachemire, de chameau, la laine, le mohair, la vigogne, la fibre de yack, les fils d'araignées, et la soie. Des fibres naturelles minérales existent également et comprennent l'acier, l'inox, l'argent, l'or, et le silicate de carbone et de magnésium. Enfin, les fibres naturelles inorganiques telles l'amiante, le basalte, le carbone, le quartz et le verre existent également. Les fibres chimiques sont obtenues par traitement chimique de matières naturelles, et comptent ainsi l'acétate de cellulose, l'alginate, l'ardil, l'arlan, le casenka, le coslan, le cupro, le fibrolane, le lanital, le mérinova, le polynosique, le silcool, le triacétate de cellulose, le vicara et la viscose. Les fibres synthétiques sont obtenues à partir de matières synthétiques, provenant de la synthèse de composés chimiques. Les fibres synthétiques sont séparées en fibres synthétiques organiques et minérales. On compte parmi les fibres synthétiques organiques l'acide polylactique, le polyamide, le polyester, le chlorofibre, l'acrylique, le modacrylique, le vinylique, l'élastodiène, le vinylal, l'élasthanne (par exemple le lycra), l'aramide, le polybenzimidazole, le polypropylène, le polyéthylène, le polyphénolique, polyurée, le polyuréthane, et le textilène. Parmi les fibres synthétiques minérales, on compte la fibre de verre, la céramique, l'or, l'argent, l'aluminium, la fibre de carbone et la fibre de bore.

Par « papier » est entendu la matière fabriquée à partir de fibres cellulosiques végétales. Sous cette dénomination est entendu tout type de papier dont le papier d'amate, le papier d'Arménie, le papier bouffant, le papier bristol, le papier calque, le papier cartonné, le papier chiffon, le papier de Chine, le papier chinois, le papier à cigarette, le papier coréen, le papier couché, le papier crépon, le papier à en-tête, le papier flash, le papier ganpi, le papier glassine, le papier japon, le papier joseph, le papier journal, le papier kraft, le papier lentille, le papier mâché, le papier marbré, le papier millimétré, le papier non acide, le papier bible, le papier gommé, le papier hollande, le papier peint, le papier de pierre, le papier de riz, le papier de soie, le papier sulfurisé, le papier toilette, le torinokogami, le papier de tournesol, le papier vélin, le papier vergé, le papier de verre, et le papier Whatman.

De manière préférée selon l'invention, ledit matériel biologique à stocker contient un acide nucléique, ADN ou ARN, d'origine animale, de préférence humaine, végétale, bactérienne, plasmidique, ou virale.

De manière préférée selon l'invention, ledit récipient (1) est revêtu d'un couvercle (2), de préférence constitué du même matériau que le récipient (1), permettant de rendre l'ensemble étanche à l'air et à l'eau, ledit couvercle (2) comprenant de préférence un joint renforçant l'étanchéité.

Par « joint renforçant l'étanchéité », on entend un dispositif évitant les fuites de fluide (liquide ou gaz) entre un milieu intérieur et un milieu extérieur, et pouvant être constitué de cuir, d'étoupe, de fibrine, de feutre, de caoutchouc, d'élastomères, de polymères synthétique souple, ou de métal.

Un dispositif pour le stockage à température ambiante de matériel biologique peut également comprendre un assemblage solidaire (4) d'au moins 2, de préférence un multiple de 8, de manière plus préférée 96, 384, ou 1536 dispositifs décrits précédemment.

Par « assemblage solidaire » est entendu une pluralité de dispositifs selon l'invention, rendus solidaires entre eux pour en former un assemblage.

De manière préférée selon l'invention, les récipients (1) sont reliés 2 à 2 entre eux par une tige sécable (6) permettant de détacher un ou plusieurs récipients (1) de l'ensemble.

Par « tige sécable » est entendu une tige permettant de désolidariser un ou plusieurs dispositifs de l'ensemble en coupant ladite tige, par pression ou à l'aide d'un objet permettant de les couper.

De manière préférée selon l'invention, chacun desdits récipients (1) de l'assemblage (4) est revêtu d'un couvercle (2) qui lui est solidaire permettant de rendre chacun desdits récipients (1) de l'assemblage étanche à l'air et à l'eau, de préférence lesdits couvercles (2) étant reliés deux à deux entre eux par des tiges sécables (6).

De manière préférée selon l'invention, ledit couvercle (2) comprend un joint permettant de renforcer l'étanchéité du récipient (1).

Un dispositif pour l'archivage à température ambiante de matériels biologiques, peut accueillir au moins 2 assemblages solidaires, de préférence entre 2 et 50, de manière encore plus préférée entre 2 et 10, ledit dispositif d' archivage pouvant être ouvert ou fermé par une fermeture (8) le rendant étanche à l'air et à l'eau.

De manière préférée, ledit dispositif d'archivage comprend un système (9) permettant de faire le vide d'air une fois fermé.

De manière préférée, ledit dispositif d'archivage comprend des glissières (10) permettant de maintenir lesdits assemblages (4) selon l'invention dans ledit dispositif d'archivage.

De manière préférée, chacun desdits récipients (1), assemblages (4) et/ou dispositifs d'archivages contient un identifiant unique passif, de préférence un identifiant par code-barres bi- ou tridimensionnelle à haute densité, de type Datamatrix 2D ou 3D, et/ou un identifiant unique actif, de préférence une puce basée sur la technologie d'identification par radio fréquence (RFID).

Selon un autre objet, la présente invention concerne une méthode de stockage de matériel biologique comprenant les étapes de :
a. Obtenir du matériel biologique contenant de l'acide nucléique ;
b. Disposer un support sur le moyen permettant de maintenir ledit support au-dessus et séparé du fond (5) du récipient (1) dans le dispositif de stockage selon l'invention ;
c. Disposer manuellement ou au moyen d'un automate le matériel biologique sur le support selon l'invention.

De manière préférée selon l'invention, ladite méthode de stockage comprend en plus une étape d. de :
d. Refermer le dispositif de stockage selon l'invention à l'aide dudit couvercle (2).

De manière préférée selon l'invention, ladite méthode de stockage comprend en plus les étapes de :
e. Insérer le dispositif de stockage contenant ledit matériel biologique, ou l'assemblage dont il fait partie, dans le dispositif d'archivage ;
f. Refermer ledit dispositif d'archivage selon l'invention.

De manière préférée selon l'invention, ladite méthode de stockage comprend en plus une étape g. de :
g. Faire le vide d'air du dispositif d'archivage selon l'invention.

Une méthode de désarchivage de matériel biologique peut comprendre les étapes de :
a. Déterminer quel matériel biologique est à désarchiver ;
b. Identifier par l'intermédiaire d'un lecteur d'identifiant actif la localisation dudit matériel biologique à désarchiver à l'aide de l'identifiant unique actif selon l'invention présent sur le dispositif d'archivage dans lequel il est archivé ;
c. Ouvrir le dispositif d'archivage selon l'invention dans lequel ledit matériel biologique a été identifié ;
d. Identifier, à l'aide des identifiants uniques passifs ou actifs selon l'invention, l'assemblage (4) contenant ledit matériel biologique à désarchiver ;
e. Identifier, à l'aide des identifiants uniques passifs ou actifs selon l'invention, ledit dispositif de stockage contenant ledit matériel biologique à désarchiver ;
f. Détacher le dispositif de stockage contenant ledit matériel biologique à désarchiver en sectionnant les tiges sécables (6), de préférence par simple pression ou découpage ;
g. Sortir le matériel biologique à désarchiver contenu dans ledit dispositif de stockage manuellement ou à l'aide d'un automate.

D'autres caractéristiques et avantages de l'invention apparaîtront encore à la lecture de la description qui suit de modes de réalisation préférés non limitatifs de l'invention en référence aux figures ci-jointes parmi lesquelles :
- La figure 1 représente le dispositif de stockage, en vue latérale (A), en vue de dessus (B), en vue de dessous (C) et en vue latérale intérieure (D) ;
- La figure 2 représente un assemblage de 96 dispositifs de stockage, en vue de dessus ;
- La figure 3 représente un assemblage de 96 dispositifs de stockage, en vue latérale ;
- La figure 4 représente le dispositif d'archivage en vue de face (A) et vue arrière (B) ;
- La figure 5 représente le dispositif d'archivage en vue intérieure de face.

### Exemple 1 : Archivage de matériel biologique

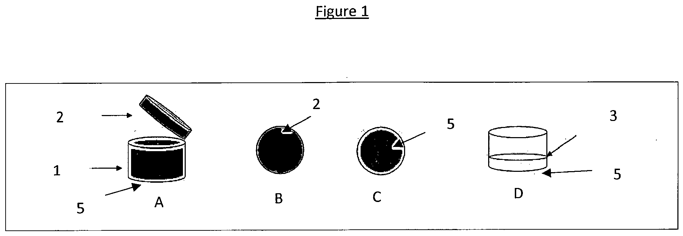

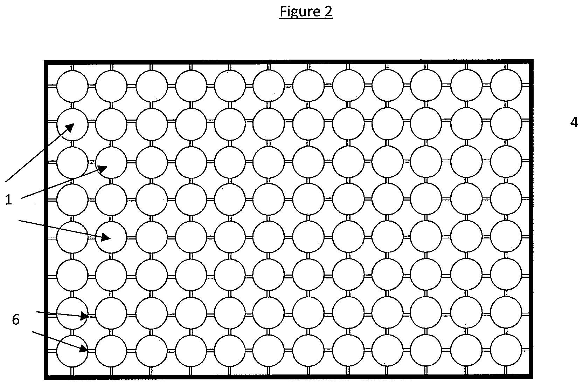

En références aux figures, le dispositif de stockage comprend, tel qu'indiqué sur la Figure 1, un récipient 1, un couvercle 2, un moyen permettant de maintenir au-dessus et séparé du fond (5) du récipient (1) un support susceptible de contenir ledit matériel biologique à stocker, afin d'empêcher le transfert de matières biologiques avec le fond du dispositif. L'utilisation selon l'invention de ce dispositif consiste à disposer au fond du récipient 1, pouvant faire partie d'un assemblage (4), par exemple de 96 récipients (1) telle que sur la Figure 2, un échantillon biologique présent dans un support, pouvant être par exemple un réactif, tel que les réactifs DNAStable^{®}/DNAStable^{®} LD ou GenTegra-DNA^{®}, ou un disque de papier FTA^{®} ou un disque de papier FTA^{®} Elute (ou équivalent) de diamètre optimisé par rapport au diamètre du dispositif de stockage selon l'invention, et pouvant être maintenu au-dessus du fond du dispositif selon l'invention par un support (3) pouvant prendre par exemple la forme d'un anneau, tel que représenté sur la Figure 1.

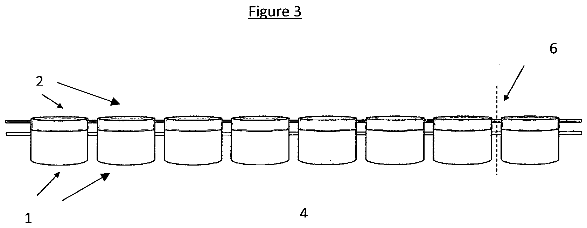

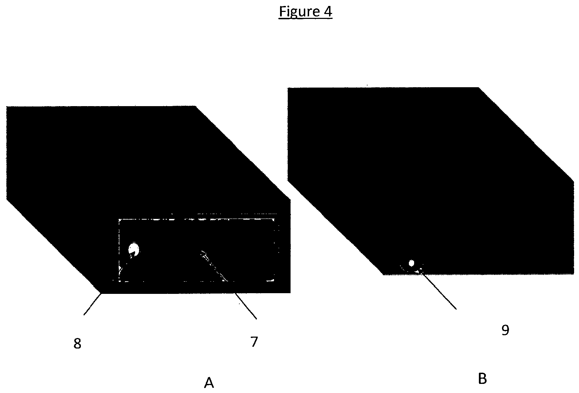

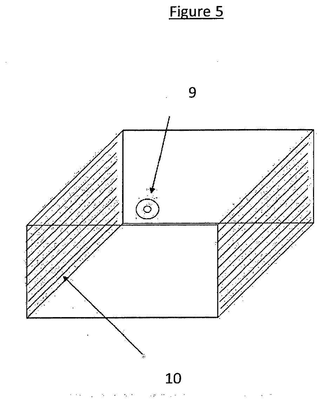

Le matériel biologique à conserver est ainsi déposé manuellement ou au moyen d'un automate sur un disque de papier FTA^{®} préalablement positionné dans un dispositif de stockage selon l'invention, présent dans un assemblage de 96, sur le moyen (3) selon l'invention permettant de le maintenir au-dessus du fond 5 du récipient 1 du dispositif selon l'invention. Un ensemble de couvercles (2) est ensuite disposé manuellement ou au moyen d'un automate sur lesdits récipients (1) de l'assemblage (4), tel qu'illustré sur la figure 3, rendant solidaire et hermétique l'assemblage (4) de récipient (1) de dispositif de stockage selon l'invention, tel qu'illustré selon la Figure 3. Cet assemblage (4) est ainsi stocké sans délai dans le dispositif d'archivage tel qu'illustré dans la Figure 4, en le faisant glisser dans celui-ci grâce aux glissières de rangement (10) (figure 5), permettant d'optimiser le classement des assemblages (4) dans le dispositif d'archivage. La porte (7) de ce dispositif d'archivage est ensuite fermée grâce au système d'ouverture/fermeture (8), rendant le dispositif d'archivage étanche à l'air et à l'eau ainsi que les assemblages (4), une fois le vide d'air réalisé au moyen (9) mis en place selon l'invention. Dans la version illustrée ici, le dispositif d'archivage permet ainsi de stocker dans un environnement anhydre et anoxique 960 échantillons (96* 10). Afin d'assurer la traçabilité du matériel biologique archivé, chaque dispositif d'archivage possède un identifiant unique passif de type Datamatrix 2D ou 3D ainsi qu'un identifiant unique actif de type puce RFID. Les données de stockage et d'identification sont ainsi renseignées afin de pouvoir procéder au désarchivage quand nécessaire, par l'intermédiaire d'un lecteur d'identifiant actif, sans nécessité d'ouvrir les autres dispositifs et risquer une dégradation du matériel biologique stocké. Dans cette configuration, il est ainsi possible de stocker environ 924 dispositifs de stockage par m² sur une hauteur d'environ 30 cm soit près de 6 000 000 d'échantillons pour 2 m³ (1 m² de surface au sol et 2 m de haut). Chacun des assemblages et des dispositifs de stockage sont également identifiés au moyen d'un identifiant unique passif de type Datamatrix 2D ou 3D et/ou un identifiant unique actif de type puce RFID, permettant également à l'aide d'un lecteur d'identifiant actif, de les localiser très simplement.

### Exemple 2 : Désarchivage de matériel biologique

Quand il est nécessaire de sortir un matériel biologique de son archivage, il suffit d'identifier par l'intermédiaire d'un lecteur d'identifiant actif la localisation dudit matériel biologique à désarchiver à l'aide de l'identifiant unique actif présent sur les dispositifs d'archivage selon l'invention. Une fois le dispositif d'archivage contenant le matériel biologique d'intérêt identifié, il est nécessaire d'ouvrir grâce au système d'ouverture/fermeture (8) ledit dispositif d'archivage, et d'identifier, toujours à l'aide d'un lecteur d'identifiant actif, l'assemblage (4) contenant le matériel biologique d'intérêt parmi tous les assemblages présents dans le dispositif d'archivage. Ensuite, on procède à l'identification, toujours à l'aide d'un lecteur d'identifiant actif, du dispositif de stockage (1) contenant le matériel biologique à désarchiver parmi les 96 dispositifs de stockages (1) compris dans l'assemblage (4). Enfin, une fois ledit dispositif de stockage (1) identifié, celui-ci est détaché à l'aide des tiges sécables le reliant aux dispositifs de stockages adjacent, par simple pression où en les découpant. Le dispositif de stockage d'intérêt est ensuite ouvert, et son contenu est récupéré manuellement ou à l'aide d'un automate.

## Revendications

1. Utilisation pour le stockage à température ambiante de matériel biologique d'un dispositif comprenant un récipient (1) constitué d'un fond (5) et d'une paroi latérale, ledit récipient (1) comprenant sur sa paroi latérale interne un moyen permettant de maintenir au-dessus et séparé du fond (5) du récipient (1) un support susceptible de contenir ledit matériel biologique à stocker, ledit moyen permettant de maintenir le support susceptible de contenir ledit matériel biologique à stocker au-dessus et séparé du fond (5) du récipient (1) étant **caractérisé en ce qu'**il est limité à un anneau (3), ledit anneau (3) étant en métal ou en plastique et étant moulé avec le récipient (1).

2. Utilisation selon la revendication 1, **caractérisé en ce que** ledit récipient (1) est de forme cylindrique.

3. Utilisation selon l'une quelconque des revendications 1 et 2, **caractérisé en ce que** ledit récipient (1) est en plastique ou en métal.

4. Utilisation selon l'une quelconque des revendications précédentes, **caractérisé en ce que** ledit récipient (1) est revêtu d'un couvercle (2), de préférence constitué du même matériau que le récipient (1), permettant de rendre l'ensemble étanche à l'air et à l'eau, ledit couvercle (2) comprenant de préférence un joint renforçant l'étanchéité.

5. Utilisation selon l'une quelconque des revendications précédentes, comprenant un assemblage solidaire (4) d'au moins 2, de préférence un multiple de 8, de manière plus préférée 96, 384, ou 1536 dispositifs pour le stockage à température ambiante de matériel biologique.

6. Utilisation selon la revendication 5, **caractérisé en ce que** les récipients (1) sont reliés 2 à 2 entre eux par une tige sécable (6) permettant de détacher un ou plusieurs récipient (1) de l'ensemble.

7. Utilisation selon l'une quelconque des revendications 5 et 6, **caractérisé en ce que** chacun desdits récipients (1) de l'assemblage (4) est revêtu d'un couvercle (2) qui lui est solidaire permettant de rendre chacun desdits récipients (1) de l'assemblage (4) étanche à l'air et à l'eau, de préférence lesdits couvercles (2) étant reliés deux à deux entre eux par des tiges sécables (6).

8. Utilisation selon la revendication 7, **caractérisé en ce que** ledit couvercle (2) comprend un joint permettant de renforcer l'étanchéité du récipient (1).

9. Utilisation selon l'une quelconque des revendications précédentes, **caractérisé en ce que** chacun desdits récipients (1) et/ou assemblages (4) contient un identifiant unique passif, de préférence un identifiant par code-barres bi- ou tridimensionnelle à haute densité, de type Datamatrix 2D ou 3D, et/ou un identifiant unique actif, de préférence une puce basée sur la technologie d'identification par radio fréquence (RFID).

10. Méthode de stockage de matériel biologique comprenant les étapes de :
a. Obtenir du matériel biologique contenant de l'acide nucléique ;
b. Disposer un support dans un dispositif pour le stockage à température ambiante de matériel biologique, comprenant au moins un récipient (1) constitué d'un fond (5) et d'une paroi latérale, ledit récipient (1) comprenant sur sa paroi latérale interne un moyen permettant de maintenir au-dessus et séparé du fond (5) du récipient (1) un support susceptible de contenir ledit matériel biologique à stocker, ledit moyen permettant de maintenir le support susceptible de contenir ledit matériel biologique à stocker au-dessus et séparé du fond (5) du récipient (1) étant limité à un anneau (3), ledit anneau (3) étant en métal ou en plastique et étant moulé avec le récipient (1), et ledit support étant disposé sur le moyen permettant de maintenir ledit support au-dessus et séparé du fond (5) du récipient (1) ;
c. Disposer manuellement ou au moyen d'un automate le matériel biologique sur ledit support.

11. Méthode de stockage de matériel biologique selon la revendication précédente, comprenant en plus une étape d. de :
d. Refermer le dispositif de stockage à l'aide d'un couvercle (2).

## Patentansprüche

1. Verwendung einer Vorrichtung für die Lagerung von biologischem Material bei Umgebungstemperatur, umfassend einen Behälter (1), der aus einem Boden (5) und einer Seitenwand besteht, wobei der Behälter (1) an seiner inneren Seitenwand ein Mittel umfasst, das es ermöglicht, einen Träger, der geeignet ist, das zu lagernde biologische Material zu enthalten, über und getrennt von dem Boden (5) des Behälters (1) zu halten, wobei das Mittel, das es ermöglicht, den Träger, der geeignet ist, das zu lagernde biologische Material zu enthalten, über und getrennt von dem Boden (5) des Behälters (1) zu halten, **dadurch gekennzeichnet ist, dass** es auf einen Ring (3) beschränkt ist, wobei der Ring (3) aus Metall oder aus Kunststoff ist und mit dem Behälter (1) geformt ist.

2. Verwendung nach Anspruch 1, **dadurch gekennzeichnet, dass** der Behälter (1) eine zylindrische Form besitzt.

3. Verwendung nach einem der Ansprüche 1 und 2, **dadurch gekennzeichnet, dass** der Behälter (1) aus Kunststoff oder aus Metall ist.

4. Verwendung nach einem der vorhergehenden Ansprüche, **dadurch gekennzeichnet, dass** der Behälter (1) mit einem Deckel (2) versehen ist, der vorzugsweise aus dem gleichen Material wie der Behälter (1) besteht, wobei es ermöglicht wird, die Anordnung wasser- und luftdicht zu machen, wobei der Deckel (2) vorzugsweise eine Abdichtung umfasst, die die Dichtheit verstärkt.

5. Verwendung nach einem der vorhergehenden Ansprüche, umfassend eine fest verbundene Anordnung (4) von mindestens 2, vorzugsweise einem Vielfachen von 8, stärker bevorzugt 96, 384 oder 1536 Vorrichtungen für die Lagerung von biologischem Material bei Umgebungstemperatur.

6. Verwendung nach Anspruch 5, **dadurch gekennzeichnet, dass** die Behälter (1) paarweise untereinander durch eine teilbare Stange (6) miteinander verbunden sind, die es ermöglicht, einen oder mehrere Behälter (1) von der Anordnung zu lösen.

7. Verwendung nach einem der Ansprüche 5 und 6, **dadurch gekennzeichnet, dass** jeder der Behälter (1) der Anordnung (4) mit einem Deckel (2) versehen ist, der fest mit ihm verbunden ist, wobei es ermöglicht wird, jeden der Behälter (1) der Anordnung (4) luft- und wasserdicht zu machen, vorzugweise wobei die Deckel (2) paarweise untereinander durch teilbare Stangen (6) verbunden sind.

8. Verwendung nach Anspruch 7, **dadurch gekennzeichnet, dass** der Deckel (2) eine Abdichtung umfasst, die es ermöglicht, die Dichtheit des Behälters (1) zu verstärken.

9. Verwendung nach einem der vorhergehenden Ansprüche, **dadurch gekennzeichnet, dass** jeder der Behälter (1) und/oder Anordnungen (4) eine passive eindeutige Kennung, vorzugsweise eine zwei- oder dreidimensionale Barcodekennung hoher Dichte einer 2D- oder 3D-Datamatrixart, und/oder eine aktive eindeutige Kennung enthält, vorzugsweise einen Chip, der auf der Hochfrequenzidentifikationstechnologie (RFID) basiert.

10. Verfahren zum Lagern von biologischem Material, umfassend die folgenden Schritte:
a. Erhalten des biologischen Materials, das Nukleinsäure enthält;
b. Anordnen eines Trägers in einer Vorrichtung für die Lagerung von biologischem Material bei Umgebungstemperatur, umfassend mindestens einen Behälter (1), der aus einem Boden (5) und einer Seitenwand besteht, wobei der Behälter (1) an seiner inneren Seitenwand ein Mittel umfasst, das es ermöglicht, einen Träger, der geeignet ist, das zu lagernde biologische Material zu enthalten, über und getrennt von dem Boden (5) des Behälters (1) zu halten, wobei das Mittel, das es ermöglicht, den Träger, der geeignet ist, das zu lagernde biologische Material zu enthalten, über und getrennt von dem Boden (5) des Behälters (1) zu halten, auf einen Ring (3) beschränkt ist, wobei der Ring (3) aus Metall oder aus Kunststoff ist und mit dem Behälter (1) geformt ist und der Träger auf dem Mittel angeordnet ist, das es ermöglicht, den Träger über und getrennt von dem Boden (5) des Behälters (1) zu halten;
c. manuelles Anordnen oder Anordnen mittels eines Automaten des biologischen Materials auf dem Träger.

11. Verfahren zum Lagern von biologischem Material nach dem vorhergehenden Anspruch, umfassend ferner einen folgenden Schritt d.:
d. Verschließen der Lagervorrichtung mit Hilfe von einem Deckel (2).

## Claims

1. Use of a device for storing biological material at ambient temperature, comprising a container (1) consisting of a bottom (5) and a side wall, said container (1) comprising on its internal side wall a means for supporting above and separated from the bottom (5) of the container (1) a support capable of containing said biological material to be stored, said means for supporting the support capable of containing said biological material to be stored above and separated from the bottom (5) of the container (1) being **characterized in that** it is limited to a ring (3), said ring (3) being made of metal or plastics material and being molded with the container (1).

2. Use according to claim 1, **characterized in that** said container (1) is cylindrical.

3. Use according to any of claims 1 and 2, **characterized in that** said container (1) is made of plastics material or metal.

4. Use according to any of the preceding claims, **characterized in that** said container (1) is covered with a lid (2), preferably made of the same material as the container (1), for making the assembly airtight and watertight, said lid (2) preferably comprising a seal which reinforces the sealing.

5. Use according to any of the preceding claims, comprising an assembly (4) secured to at least 2, preferably a multiple of 8, more preferably 96, 384, or 1536 devices for storing biological material at ambient temperature.

6. Use according to claim 5, **characterized in that** the containers (1) are connected in pairs by a divisible rod (6) which detaches one or more containers (1) from the assembly.

7. Use according to any of claims 5 and 6, **characterized in that** each of said containers (1) of the assembly (4) is covered with a lid (2) which is secured thereto and makes each of said containers (1) of the assembly (4) airtight and watertight, preferably said lids (2) being connected in pairs by divisible rods (6).

8. Use according to claim 7, **characterized in that** said lid (2) comprises a seal for reinforcing the sealing of the container (1).

9. Use according to any of the preceding claims, **characterized in that** each of said containers (1) and/or assemblies (4) contains a passive unique identifier, preferably a 2D or 3D Datamatrix high density two- or three-dimensional barcode identifier, and/or an active unique identifier, preferably a chip based on radio frequency identification (RFID) technology.

10. Method for storing biological material comprising the steps of:
a. obtaining biological material containing nucleic acid;
b. arranging a support in a device for storing biological material at ambient temperature, comprising at least one container (1) consisting of a bottom (5) and a side wall, said container (1) comprising on its internal side wall a means for supporting above and separated from the bottom (5) of the container (1) a support capable of containing said biological material to be stored, said means for supporting the support capable of containing said biological material to be stored above and separated from the bottom (5) of the container (1) being limited to a ring (3), said ring (3) being made of metal or plastics material and being molded with the container (1), and said support being arranged on the means for supporting said support above and separated from the bottom (5) of the container (1);
c. arranging manually or by means of an automaton the biological material on said support.

11. Method for storing biological material according to the preceding claim, further comprising a step d. of:
d. closing the storage device using a lid (2).
